# EUROPEAN PATENT APPLICATION

(11) **EP 4 850 608 A1**
(43) Date of publication of application: **09.12.2026**
(21) Application number: 26183380.0
(22) Date of filing: 05.06.2026
(51) Int. Cl.: A61B 6/51, A61B 6/00

(54) **METHOD AND SYSTEM FOR POST-PROCESSING OF PANORAMIC DENTAL IMAGES**

(30) Priority: 06.06.2025 US 202519230921
(71) Applicant: Eichner, Stefan, 69123 Heidelberg (DE); Maur, Susanne, 64625 Bensheim (DE)
(72) Inventor: Eichner, Stefan, 69123 Heidelberg (DE); Maur, Susanne, 64625 Bensheim (DE)
(74) Representative: Aldridge, Henry Alexander

(57) **Abstract**

A computer-implemented method that performs an initial processing of a panoramic image derived from multiple x-ray projection images of a dental region of a patient. The computer-implemented method can determine from the initial processing a depth information of anatomical structures of the panoramic image. The computer-implemented method can identify at least one of: a clinically relevant region of a jaw region and one or more clinically relevant anatomical structures of the jaw region of the panoramic image. The computer-implemented method may perform a post-processing to at least one of: the clinically relevant region and the one or more clinically relevant anatomical structures using the depth information to enhance visualization of the at least one of the clinically relevant regions and the one or more clinically relevant anatomical structures of the panoramic image. The method can include generating an updated panoramic image resulting from the performing the post-processing.

## Description

### TECHNICAL FIELD OF THE INVENTION

The present application relates to computer-implemented methods and systems for panoramic dental images, and more particularly to a localized post-processing of such dental images on a region and/or structural basis.

### BACKGROUND ART OF THE INVENTION

Dentists generally use a variety of dental images generated with different imaging techniques for the purpose of diagnosis and therapeutic treatment. This includes an extraoral panoramic radiograph, an orthopantomogram ("OPG"), which provides dentists a quick overview of their patient's dental regions. These radiographs typically display all teeth in the upper and lower jaw, the nerve canal, temporomandibular joints, and portions of the eye sockets using tomosynthesis procedures for reconstruction. OPG utilizes an x-ray system for dental panoramic imaging that includes an x-ray generator and an x-ray detector that are rotated around a patient's head. The x-ray detector acquires a plurality of x-ray projections of the patient's dental regions during the rotation.

The panoramic image is reconstructed from the plurality of projection images by using reconstruction parameters which describe a layer containing the patient's dental regions that should be in focus, namely sharply depicted in the panoramic image. In general, default reconstruction parameters which describe a default layer are used to focus on the dental regions, especially the patient's jaw arch. However, regions outside of the patient's jaw arch are captured with the panoramic image and can influence the focusing of the image. Furthermore, each patient has a different jaw arch and other anatomical variations including teeth angulation. Additionally, each patient experiences a slightly different position and orientation during image acquisition. These and other factors can result in the panoramic image not being perfectly focused for the patient's jaw arch. This can make the diagnosis and treatment based on the panoramic image inaccurate and difficult.

This lack of focus has been addressed in several ways. Manual focusing and adjustment is possible but can be tedious and requires multiple exposures. Software based automatic focusing approaches have also been developed. These automatic approaches focus of the panoramic image by adjustment after the projection images are acquired.

Such automatic focusing techniques attempt to automatically optimize the sharpness of the resulting panoramic image for a given set of projection images by adjusting the reconstruction parameters. These methods use a mathematical metric, e.g., a measure of sharpness during image processing, which can be calculated for a reconstructed panoramic image, and then optimize the value of this metric by adjusting the reconstruction parameters, often in an iterative, gradient-descent type of numerical optimization. This procedure results in panoramic images that have an optimal measure of sharpness in a mathematical sense at a global image level, but are not necessarily optimal for diagnostic use at a more localized level. This is because the structures in focus do not always correspond the specific anatomical structures that are relevant for the dentist and the patient's clinical condition. For example, structures such as dental implants can influence the focusing as the implant will dominate the possible sharpness improvement on the clinically much more relevant teeth structures close to the implant. Other more globalized anatomical structures such as nasal cavities, sinuses, bone including opposing jaw shadows and the spine can also influence the focusing in an undesired manner from a diagnostic sense.

### BRIEF SUMMARY

The present inventors have recognized that there is a need for an improved technique that allows for post-processing of an initial panoramic image that has already been subjected to automatic focusing. Thus, the present application disclose of in-depth-sharpness analysis that result from autofocus processing for the subsequent post-processing of the reconstructed panoramic image. According to one example, such post-processing is used to sharpen focus in a clinically relevant region and/or is used to sharpen focus of a anatomical structure(s). The post-processing is not only about enhancing the focus, but also includes enhancing the brightness, contrast, and/or noise of clinically relevant region(s)/structure(s). This post-processing, which is performed locally such as only to a jaw region or subregion(s) thereof and can be applied to specific relevant structure(s) or region(s) of the initial panoramic image. This methodology allows the region(s) and/or relevant structure(s) to be displayed more sharply without being disturbed by non-relevant structures.

Thus, the inventors have developed a method and system for enhanced post-processing of initial panoramic images that utilizes depth information to improve visualization of clinically relevant anatomical structures. The invention addresses limitations in conventional global post-processing approaches by enabling region-specific and/or structure-specific optimization.

The system and method performs global image processing using automatic focusing process to obtain depth information for anatomical structures within the initial panoramic image, then identifies relevant region(s) and/or structure(s) within the jaw region using the depth information. Using this depth information, the system and method can perform region-specific and/or structure-specific post-processing to enhance visualization of relevant anatomical structures while suppressing or excluding less clinically relevant regions. The term "suppressing" means a decrease in brightness/sharpness of less relevant structures in addition to the normal post-processing and/or the enhancement of clinical relevant structures. Terms "automatic focusing process" or "automatic focusing" as used herein mean the process of automatically adjusting the focus of an image or plurality of images to achieve sharper image or images or improve the clarity of the image or images. This process is done with software implemented systems and methods by evaluating the sharpness or clarity of the image or images and adjusting a focal position to achieve a better contrast.

Thus, according to one example, the system and method use derived depth information of sharp structures of the autofocus result (referring to the derived optimal depth position per patch (e.g. by selecting the maximum in the course of the sharpness)) for: A) normalization / noise reduction per pixel depending on the depth position, optional color representation per pixel for the visualization of depth position (providing positional information of object for the user) and/or B) use of the entire course of the sharpness in depth (3D information) as basis for object segmentation (e.g. in terms of high-contrast structures, low-contrast structures, size of the structures). The latter can be used to suppress or emphasize corresponding region-specific areas within the reconstructed panoramic image and optional color representation regarding the extent of objects (optical separation of objects for the user). For B) above, the 3D data space (3D information) is the basis for the adaptive post-processing of the reconstructed panoramic image. A (sharpness) value is used to determine the optimal depth per autofocus patch based on the courses of sharpness. This reduces the 3D data space to one optimal depth value per patch (a depth map). For A) above, the depth map is the basis for adaptive post-processing of the reconstructed panoramic image. This depth map is also the basis for the reconstruction of the depth-optimized (autofocused) panoramic image. After the reconstruction, conventional and new autofocus based post-processing can also take place.

Relevant anatomical structures (also called clinically relevant anatomical structures herein) include, but are not limited to the teeth, particular regions of the teeth including root canals, root tips, tooth crown, tooth pulps and the nerve canals running along the jawbone. Unless otherwise specified herein, the term "region" and similar terms such as "clinically relevant region" or "relevant region" is used to describe an area of the panoramic image that contains two or more relevant anatomical structures. Less clinically relevant regions include, but are not limited to, dental replacements (e.g., dental crowns, braces, veneers, bridges), nasal cavities, sinuses, bone (particularly bone on an outer portion of the jaw region or other areas of the skull outside the jaw region), an opposing jaw shadow and the spine. More particularly, the method and system enhance relevant dental structure(s) and/or region(s) by utilizing position information derived from depth data, rather than relying solely on grey values which cannot effectively distinguish between different anatomical structures.

The present application overcomes the disadvantages of the prior art including the reliance on grey values, which can lead to clinically relevant region(s) or structure(s) being out of focus. Thus, advantages of the present application include improved diagnostic capabilities through optimized display of clinically relevant dental area(s) and/or clinically relevant dental structure(s), reduction of irrelevant image areas, and localized optimization of sharpness, contrast, and noise reduction for the panoramic image in the clinically relevant dental area(s) and/or clinically relevant dental structure(s). The system enables a more patient-specific optimization by adapting post-processing parameters based on individual anatomical variations. Post-processing parameters are (e.g., the brightness, contrast or gamma value or defined data window (min and max) value to be emphasized, the amount of noise reduction, the filter size used, etc.). The present application results in shorter diagnosis times due to visual focusing on clinically relevant areas within the panoramic image, while effectively suppressing artifacts and less relevant regions such as nasal regions, and spine artifacts. The present application represents an advancement over conventional global post-processing methods by enabling targeted enhancement of clinically relevant structures on a region-by-region or structure-by-structure basis, leading to improved image quality and diagnostic efficiency.

Advantages listed herein are only examples and are not intended to be limiting to the illustrative examples. Additional or different advantages can be realized by specific illustrative examples. Furthermore, a particular illustrative example can have some, all, or none of the advantages listed above or below.

### BRIEF DESCRIPTION OF THE DRAWINGS

To easily identify the discussion of any particular element or act, the most significant digit or digits in a reference number refer to the figure number in which that element is first introduced.
FIG. 1 depicts a block diagram of a network of data processing systems in accordance with an illustrative example.
FIG. 2 shows a schematic diagram of an extraoral x-ray system in accordance with an illustrative example.
FIG. 3 is a schematic view from the top of the x-ray system of FIG. 2 during operation in accordance with an illustrative example.
FIG. 4 is an initial panoramic image generated by the x-ray system having undergone initial image processing in accordance with an illustrative example.
FIG. 5 is a projection region showing some automatic focus layer meshes having different depths in accordance with an illustrative example
FIG. 6 is the panoramic image of FIG. 4 having undergone a first post-processing in accordance with an illustrative example.
FIG. 7 is the panoramic image of FIG. 4 having undergone a second post-processing in accordance with an illustrative example.
FIG. 8 is the panoramic image of FIG. 4 having undergone both the first post-processing and the second post-processing in accordance with an illustrative example.
FIG. 9 is a portion of a jaw region of another panoramic image having undergone post-processing of a clinically relevant region and clinically relevant anatomical structures using depth information derived from the initial processing of FIGS. 4 and 5 in accordance with an illustrative example.
FIG. 10 is a flow diagram of a method for processing a panoramic image in accordance with an illustrative example.

### DETAILED DESCRIPTION OF THE EXAMPLES

The method and systems according to the invention, which is explained in detail below, are computer-implemented, and can be carried out on a computerized dental imaging devices and/or other electronic devices.

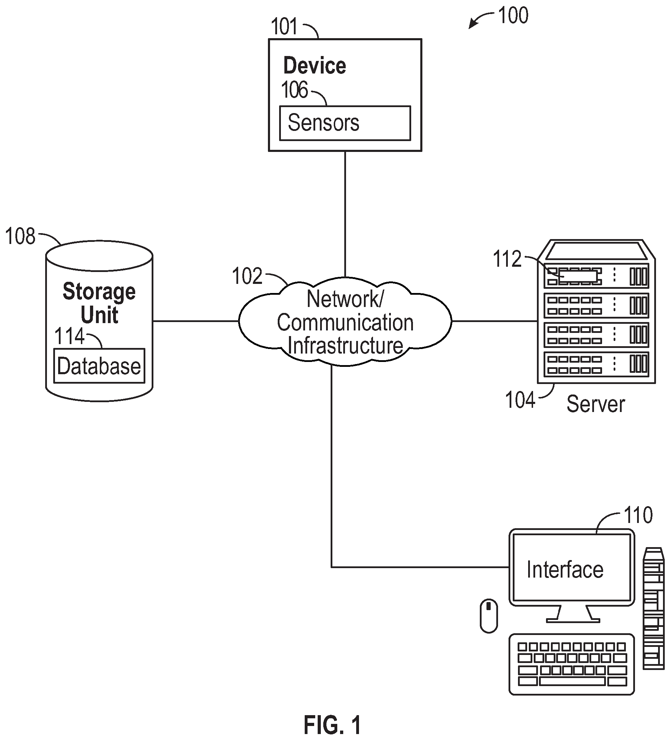

FIG. 1 depicts a block diagram of a system 100 in which illustrative examples can be implemented. System 100 is a network of electronically implemented components in which the illustrative examples can be implemented. System 100 can include network/communication infrastructure 102. Network/communication infrastructure 102 can be the medium used to provide communications links between various devices, databases and computers connected together within the system 100. Network/communication infrastructure 102 can include connections, such as wired connections, wireless communication protocols, or other suitable data connectivity. System 100 can be implemented with use of the Internet. Network/communication infrastructure 102 can represent a collection of networks and gateways that use the Transmission Control Protocol/Internet Protocol (TCP/IP) and other protocols to communicate with one another.

The x-ray device 118 can implement examples described herein. The x-ray device 118 can include a local engine 116 which can further include an embedded application to use data of the x-ray device 118 for performing routines such as for calibration, operation, etc. The embedded application can also execute in any of data processing systems (server 104 or processor 110), such as client server application 112 in server 104 according to other examples.

Processors and/or servers are purely exemplary of certain data processing systems connected to network/communication infrastructure 102 and are not intended to exclude other configurations or roles or components for these data processing systems. The server 104 can be coupled to the network/communication infrastructure 102 along with the storage unit 108. Software applications can execute on any computer in the system 100. The processor 110 is also coupled to network/communication infrastructure 102. A data processing system, such as the server 104 in addition to the processor 110 can include data and can have software applications or software tools executing thereon.

FIG. 1 depicts certain components that are usable in an example implementation of an example. As another example, an example can be distributed across several data processing systems and a data network as shown, whereas another example can be implemented on a single data processing system within the scope of the illustrative examples. It should be noted that the initial processing (e.g., automatic focusing process) and post-processing need not be gathered using the components of the system 100 such as the x-ray device 118. For example, raw panoramic images from another source can be supplied via the network/communication infrastructure 102 such as to the server 104, storage unit 108, processor 110, etc. for processing by the system 100. FIG. 1 is intended as an example, and not as an architectural limitation for the different illustrative examples.

Components of the system such as the processor 110, the server 104, and the storage unit 108 can include a computer readable storage medium (or media) that are non-transitory having computer readable program instructions thereon for causing a processor to carry out aspects of the present invention. This can include a database 114. The computer readable storage medium can be a tangible device that can retain and store instructions for use by an instruction execution device. The computer readable storage medium can be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer readable storage medium includes the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon, and any suitable combination of the foregoing.

Computer readable program instructions described herein are non-transitory and can be downloaded to respective computing/processing devices from a computer readable storage medium or to an external computer or external storage device via a network, for example, the Internet, a local area network, a wide area network and/or a wireless network. The network can comprise copper transmission cables, optical transmission fibers, wireless transmission, routers, firewalls, switches, gateway computers and/or edge servers. A network adapter card or network interface in each computing/processing device receives computer readable program instructions from the network and forwards the computer readable program instructions for storage in a computer readable storage medium within the respective computing/processing device. The computer readable program instructions can be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. These computer readable program instructions can also be stored in a computer readable storage medium that can direct a computer, a programmable data processing apparatus, and/or other devices to function in a particular manner, such that the computer readable storage medium having instructions stored therein comprises an article of manufacture including instructions which implement aspects of the function/act specified in the flowchart and/or block diagram block or block. The computer readable program instructions can also be loaded onto a computer, other programmable data processing apparatus, or other device to cause a series of operational steps to be performed on the computer, other programmable apparatus or other device to produce a computer implemented process, such that the instructions which execute on the computer, other programmable apparatus, or other device implement the functions/acts specified in the flowchart and/or block diagram block or blocks.

The system 100 can include additional servers, clients, and other devices that are not shown. Server 104 includes the server application 112 that can be configured to implement one or more of the functions described herein for displaying restoration proposals in accordance with one or more examples.

The system 100 can be used for implementing a client-server environment in which the illustrative examples can be implemented. A client-server environment enables software applications and data to be distributed across a network such that an application functions by using the interactivity between a client data processing system and a server data processing system. The system 100 can employ a service-oriented architecture where interoperable software components distributed across a network can be packaged together as coherent business applications. The system 100 can take the form of a cloud, and employ a cloud computing model of service delivery for enabling convenient, on-demand network access to a shared pool of configurable computing resources (e.g. networks, network bandwidth, servers, processing, memory, storage, applications, virtual machines, and services) that can be rapidly provisioned and released with minimal management effort or interaction with a provider of the service.

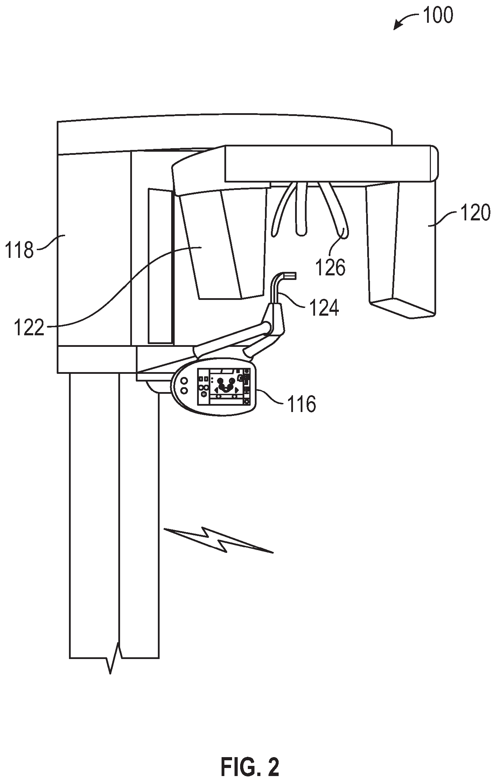

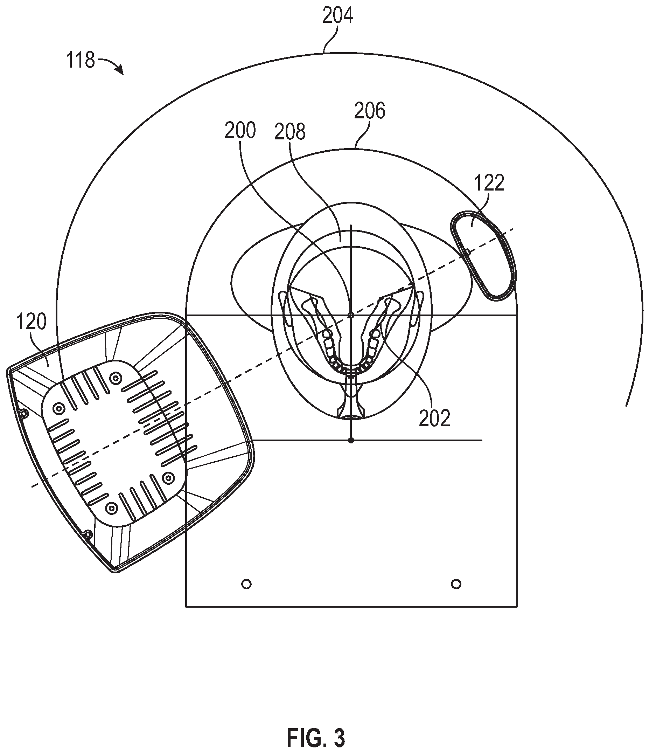

As will be further described in FIGS. 2 and 3, x-ray radiography can be performed by positioning an x-ray source on one side of a patient (e.g., a jaw region of a patient) and causing the x-ray source to emit x-rays through the volume and toward an x-ray detector located on the other side of the patient. As the x-rays pass through the patient and a volume from the x-ray source. The x-rays energies are absorbed to varying degrees depending on the composition of the volume and anatomy of the patient, and the x-rays arrive at the x-ray detector. This process forms a two-dimensional x-ray image or projection image (also known as a radiograph) based on the cumulative absorption through the volume. This series of two-dimensional images is combined to form a panoramic image as known in the art.

FIG. 2 shows the x-ray device 118 of the system 100 that can be used for performing patient imaging and for generating the panoramic images discussed herein according to some examples. The x-ray device 118 has an x-ray source 120 and x-ray detector 122, which are rotated around the patient's head during the recording. The trajectory of the x-ray source 120 and x-ray detector 122 during the recording can describe a circular path or non-circular path having an approximately known positioning relative to one another. The patient's head is positioned in the x-ray device 118 with the bite 124 and the head fixation 126. The system 100 can include the local engine 116 such as an operating control. This local engine 116 can have computing system in some cases for control of the x-ray device 118, for a display, etc. The x-ray device 118 can be electronically connected to the various components of the system 100 as previously discussed.

FIG. 3 schematically depicts an operation of an x-ray device 118 in accordance with an illustrative example. The x-ray device 118 comprises the x-ray detector 122, and the x-ray source 120. The x-ray device 118 can have a plurality of rotation centers 200. The rotation center 200 is a center or reference point for a focal layer 202. The x-ray source 120 and the x-ray detector 122 can move for x-ray acquisition. The x-ray source 120 and x-ray detector 122 have a known spacing relative to one another and move along a trajectory (a source trajectory 204 and a detector trajectory 206) for the rotation centers 200. The rotation centers 200 can be predefined such that the source trajectory 204 and the detector trajectory 206 have a known geometric relationship. X-ray beams are projected while the x-ray source 120 and the x-ray detector 122 rotate around a patient's head 208, in particular, with respect to the focal layer 202 of the patient's head 208.

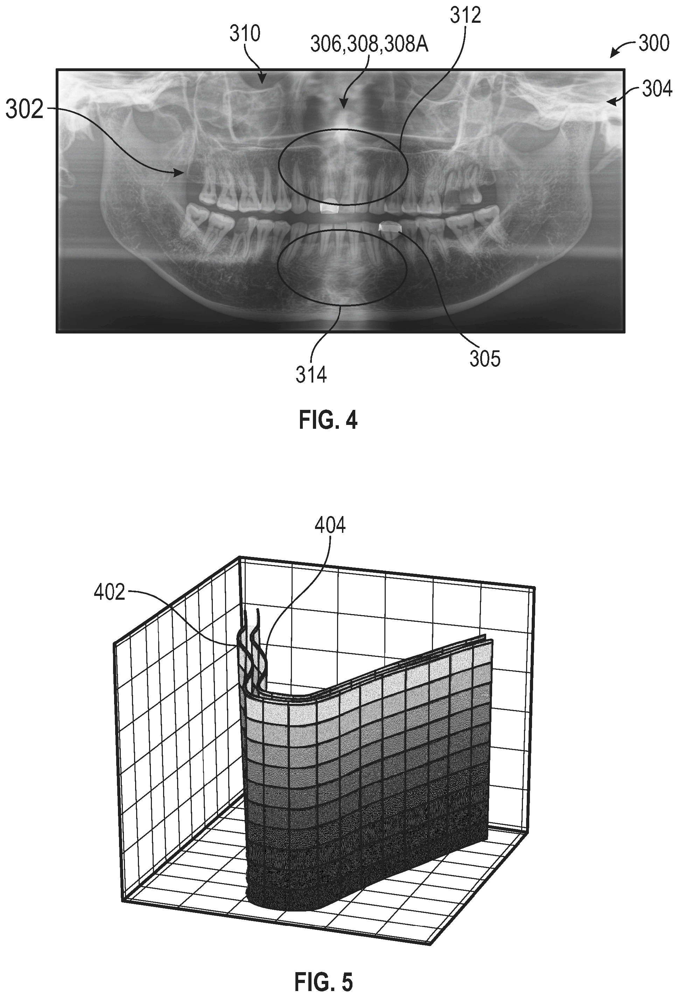

FIG. 4 shows an initial panoramic image 300 of a patient's dental anatomy. The panoramic image 300 illustrates all the teeth present in the upper and the lower jaw, the nerve canal, as well as the temporomandibular joints and part of the eye sockets. The panoramic image 300 in FIG. 4 has undergone an initial processing according to the methods, systems and techniques discussed herein whereby depth information regarding the patient's dental anatomy is obtained such as from raw projection images. Such processing can include applying an automatic focusing process via software or other means to raw projection images including the jaw region 302 and the peripheral region 304 with the intent of reconstruction of the patient's dental anatomy so as to obtain the initial panoramic image 300 shown. Autofocus analysis is done only once during the reconstruction of the panoramic image and before the post-processing of the reconstructed panoramic image. Autofocus analysis is a patch-based approach (with/without overlapping of the autofocus patches) along the depth of panoramic layers (alternate focal curve positions in depth). The patches are image regions (1 pixel or larger) for which a sharpness value of displayed structures is calculated. For each autofocus patch, a sharpness value is determined over the depth, so over the panoramic layers a course of sharpness is produced. A sharpness value can be calculated from the image gradient, the L2 norm or the L1 norm in the image region. Initial processing can include inversion of the brightness of the panoramic image in addition to automatic focusing process which can include a generalized (whole image or substantially whole image) contrast enhancement and a noise reduction to achieve a good image impression. According to some examples, initial processing can be used to achieve good image impression for the panoramic image 300 which can be shown to the dentist. According to some examples, initial processing may include inversion, contrast enhancement and/or noise reduction. The initial processing includes capturing a three-dimensional positioning of various anatomical structures of the panoramic image 300. Autofocus analysis is based on multiple focal layers which are pre-defined and also reconstructed. This is done typically without post-processing. Each focal layer has a specific position in the 3D scan geometry. Dental objects are displayed in these reconstructed focal layers differently in terms of their relative sharpness. Based on an overall analysis, reconstructed focal layers have a three dimensional information, which can be estimated/derived on a per object basis. As further shown in FIGS. 3 and 5 in combination with FIG. 4, there exists a special path curve for every available panoramic program on an extraoral x-ray device, which defines the orbit of the x-ray source 120 (FIG. 3) and the x-ray detector 122 (FIG. 3) around the patient's head 208 (FIG. 3).

FIG. 5 reproduces a data space and shows positions of two focal layers 402 and 404 (further focal layers are possible but are not illustrated in FIG. 5 for simplicity) in such data space. The selected one of the focal layers 402 or 404 defines a sharpness of the imaged anatomical structures within the panoramic image 300. The thickness of the layer in focus also has a direct influence on the representation of the objects within the panoramic image 300. This thickness is a measure of the local blurring of the objects outside (in the labial/buccal direction) of the focal layer. Each focal layer 402 and 404 is an derived focal layer based on the empirically determined focal layer that is intended to depict objects along an average focal layer of a standard patient jaw in the best possible manner. However, patient-specific variations as well as incorrect positioning occur and reduce the image quality as well as the diagnostic capability of the panoramic image. Automatic focusing process attempts to subsequently adjust the focal layer 402 and/or 404 so that all relevant anatomical structures along the mandibular arch are in focus. The automatic focusing processes calculates the locally adapted layer position, records the imaged structures in depth (gathering depth information such as in the data space and any additional information such data space contains, e.g., about the anatomical structures). This data which includes depth information can further improve the post-processing of the reconstructed panoramic raw images and can enable a structure-specific optimization of the image impression as further discussed herein. On a general level, clinically relevant anatomical structures can have a minimum or different depth/thickness as compared with other less relevant anatomical regions or structures. This can require analysis of such clinically relevant anatomical structures or region on a localized level.

As shown in FIG. 4, initial processing was not entirely successful in sharpening clinically relevant anatomical structures indicated by regions 312 and 314. These regions 312 and 314 are blurred, noisy, lack contrast and desired edge sharpness in clinically relevant regions and of clinically relevant anatomical structures due to various factors and reasons as discussed previously above including but not limited to that automatic image processes are performed globally (on whole image or substantially whole image basis) and do not use any information about the relevant image content (e.g. teeth along the jaw arch) as additional information. As a result, the panoramic image 300 has been adjusted globally and it is not possible to suppress or exclude clinically less clinically relevant regions such as dental replacements 305, nasal cavity 306, sinuses 310 and bone 308 including an opposing jaw shadow and a spine 308A. Clinically relevant region(s) and clinically relevant anatomical structures are not sharpened by post-processing in FIG. 4. This makes diagnosis and reporting more difficult, as the image content of the panoramic image 300 is only optimized with regard to the brightness on a global level, regardless of any anatomical structural information (e.g. teeth or teeth related areas v. bone, for example).

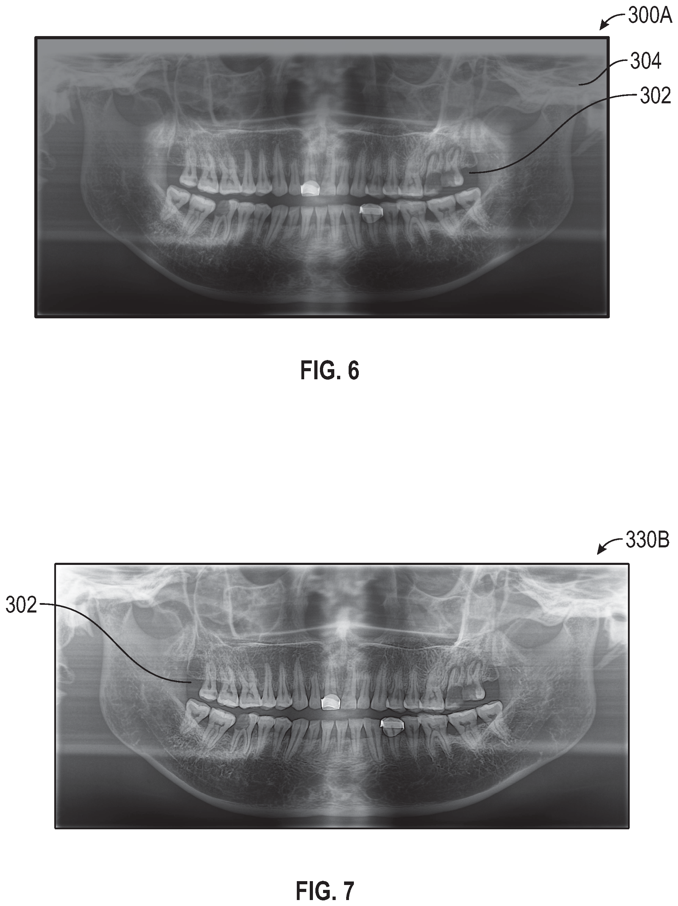

The present application enables local adaptation of post-processing to the imaged structures for special highlighting of clinically relevant structures or for suppression of clinically less relevant or irrelevant image area. This post-processing can include adjusting a focus depth, adjusting a contrast, adjusting a color information, performing a noise reduction, and adjusting a brightness based on the depth information gathered from FIGS. 2-4. Regarding contrast, contrast describes the slope in signal between signal values of low and high amplitude. The lower the contrast the more similar look the displayed signal values. To enhance (e.g., edges) the contrast/slope is increased to make enhanced feature more visible / clear. Additionally, based upon the data in the data space, depth information can include, but is not limited to grey values/absorption resolved in depth, differences/gradients of these grey values, etc. The suppression/hiding of less relevant image areas becomes possible, as does the enhancement/highlighting of relevant image areas for the diagnosis. For example, a post-processing based local contrast optimization can be performed. The brightness of marginal areas 304 of the panoramic image 300A, which are less clinically relevant, can be reduced or suppressed as shown in FIG. 6 in order to direct the dentist's focus to the areas relevant for the diagnosis at the jaw region 302. Thus, FIG. 6 shows the peripheral region 304 reduced, excluded or suppressed for post-processing and having a reduced in contrast and brightness. This reduces the influence and enhancement of less clinically relevant regions including one or more of the nasal cavities, sinuses and bone including an opposing jaw shadow and the spine.

FIG. 7 shows a panoramic image 300B that has undergone post-processing on the jaw region 302 including one or more of contrast-enhancing a best one or more of the one or more clinically relevant anatomical structures, adjusting brightness of the jaw region depending on the depth information and performing the noise reduction based upon a size, number or relative brightness of the less clinically relevant regions of the panoramic image. Contrast enhancing means to display clinically relevant structure(s) or region(s) in an improved manner as compared with less clinically relevant structure(s) or region(s). This can include also increasing contrast in clinically relevant region(s)/structure(s) and by suppression of less clinically relevant region(s)/structure(s). Post-processing can be applied to the complete panoramic image. However, a different post-processing method, or the same post processing method with different parameters, can applied to clinically relevant region(s), structure(s) as compared with less clinically relevant region(s) or structure(s).

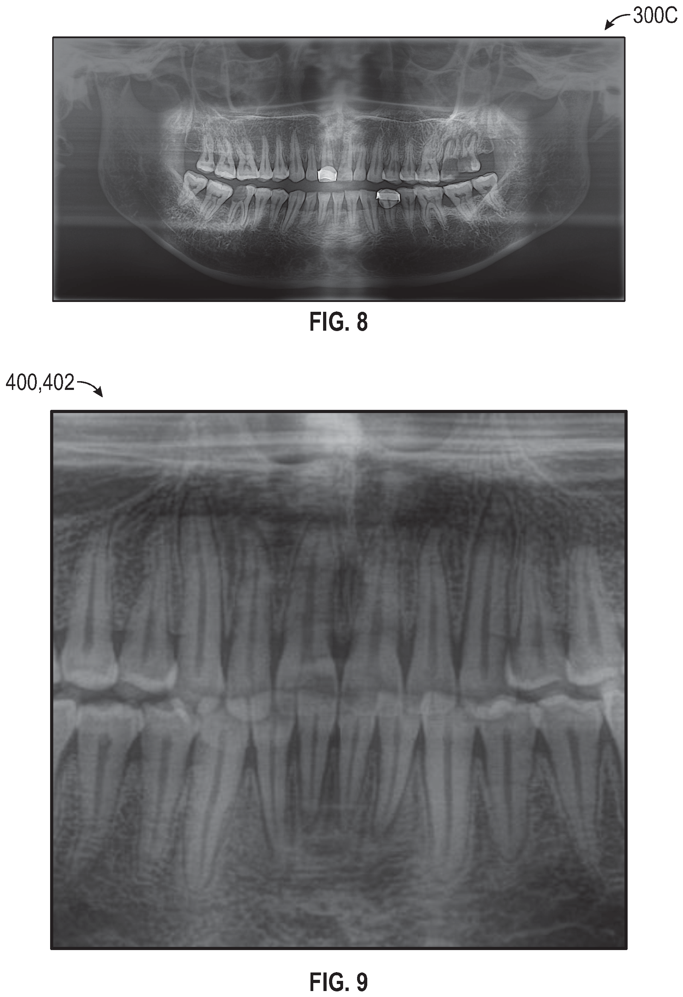

It is also possible to combine/superimpose several post-processing operations. FIG. 8 shows the post-processing of FIGS. 6 and 7 applied together on the panoramic image 300C.

FIG. 9 shows a portion of a panoramic image 400, in particular, a portion of the jaw region 402 thereof. The panoramic image 400 has undergone post-processing according to the techniques described herein to identify anatomical structures which require additional focusing to emphasize the anatomical structures. Identification can be done prior to initial processing or later during post-processing. For example, identification can be based on a region definition before the autofocus analysis. The predefined region could cover all dental regions to be improved with the techniques described herein. Identification can include use of artificial intelligence (AI) techniques, for example. These AI techniques could be also used for identifying and segmenting of clinically relevant regions before autofocus analysis, for example. Thus, FIG. 9 shows clinically relevant anatomical structures with enhanced edges including with regard to the teeth, root canals, root tips, tooth crowns, and tooth pulps as compared to similar structures in FIG. 4. Nerve canals (not shown in FIG. 9) are also considered clinically relevant anatomical structures and can be enhanced according to some examples. According to some examples, the methods and systems described can include identifying and applying various techniques described herein to a transition region between the clinically relevant region(s)/structure(s) and the non-relevant regions (e.g., less clinically relevant region(s)/structure(s)), where the parameters of post-processing or the results of the different post-processing methods are interpolated or otherwise merged or integrated).

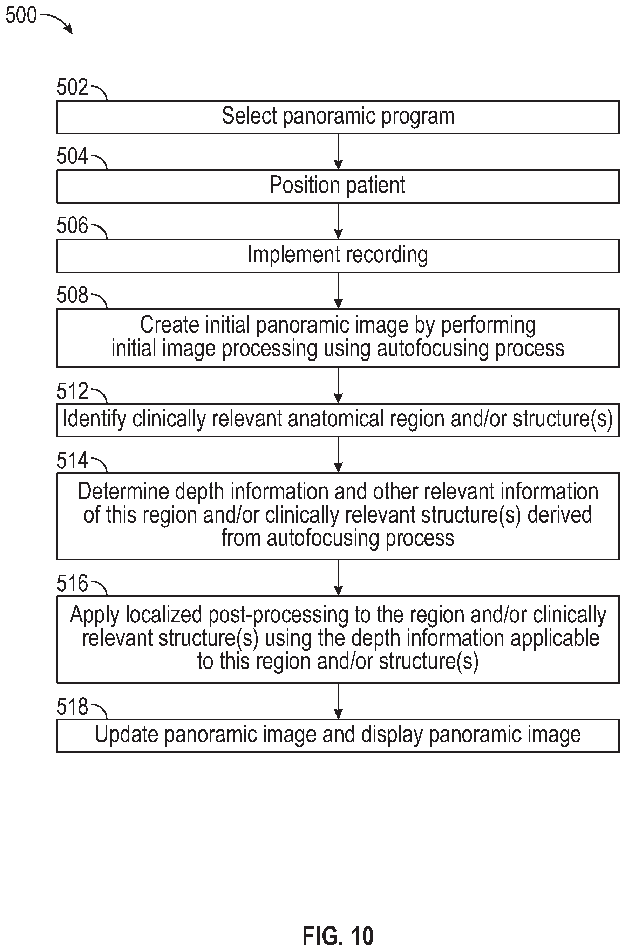

FIG. 10 is a flow diagram of a computer implemented method 500 according to one example. The method 500 can optionally include selection of a panoramic program 502, positioning of a patient 504, implementation of x-ray recording 506 and creation of an initial panoramic image 508 such as by performing an initial processing of a panoramic image derived from x-ray projection images of a patient. It should be noted that these steps are entirely optional and that initial processing and post-processing steps as discussed herein can be performed on a panoramic image gathered from another location or another source.

The method 500 can include identifying 512 clinically relevant anatomical region(s) and/or clinically relevant anatomical structures of the panoramic image using the depth information. This can include identifying at least one of the anatomical structures that requires additional focusing to emphasize the anatomical structures. The identifying 512 can additionally or alternatively include identifying less clinically relevant regions or structures. In some examples, this analysis can be done with imaging software, with trained machine learning systems or other known techniques. Such identification can also be performed manually according to some examples. It should be noted that such clinically relevant anatomical region(s) and/or clinically relevant anatomical structures may not be labeled or otherwise identified prior to the initial processing, which differs from the techniques described in WO2023110494A1, the entire disclosure of which is incorporated herein by reference. Rather, such clinically relevant anatomical region(s) and/or clinically relevant anatomical structures, clinically less relevant anatomical region(s) and/or clinically less relevant anatomical structures can be identified based upon visual appearance and/or based upon depth information or other data after the panoramic image has been initially processed.

The method 500 can include determining 514 from the initial processing a depth information of anatomical structures of the panoramic image. This can include determining depth information and other relevant information of the clinically relevant region and/or clinically relevant structure(s), clinically less relevant anatomical region(s) and/or clinically less relevant anatomical structures derived from automatic focusing process or from other initial imaging process. The method 500 can include applying 516 localized post-processing to the region and/or clinically relevant structure(s) and/or applying 516 localized post-processing to clinically less relevant anatomical region(s) and/or clinically less relevant anatomical structures using the depth information applicable to this region and/or structure(s). This can include performing the post-processing to at least one of: the clinically relevant region and the one or more clinically relevant anatomical structures using the depth information to enhance visualization of the at least one of the clinically relevant regions and the one or more clinically relevant anatomical structures of the panoramic image and/or using the depth information to reduce visualization emphasis on clinically less relevant anatomical region(s) and/or clinically less relevant anatomical structures. According to some examples, post-processing can be applied to the complete panoramic image. However, a different post-processing method, or the same post processing method with different parameters, can applied to clinically relevant region(s), structure(s) as compared with less clinically relevant region(s) or structure(s). According to some examples, the methods and systems described can include identifying and applying various techniques such as those described herein to a transition region between the clinically relevant region(s)/structure(s) and the non-relevant regions (e.g., less clinically relevant region(s)/structure(s)), where the parameters of post-processing or the results of the different post-processing methods are interpolated or otherwise merged or integrated). The method 500 can then generate the panoramic image and output (e.g., display, send for further processing, etc.) the panoramic image at step 518.

Optionally, the method 500 can further include modifying post-processing parameters based on individual anatomical structures of the patient. The one or more clinically relevant anatomical structures include teeth, root canals, root tips, tooth crowns, tooth pulps, and nerve canals. The performing of the post-processing can include one or more of adjusting a focus depth, adjusting a contrast, performing a noise reduction, and adjusting a brightness based on the depth information. The method 500 can further include suppressing less clinically relevant regions of the panoramic image, wherein the less clinically relevant regions include one or more of: dental replacements, nasal cavities, sinuses and bone including an opposing jaw shadow and a spine. Suppressing can be lowering contrast, lowering brightness, lowering sharpness or combinations of these. The performing of the initial processing can include applying an automatic focusing process to substantially an entirety of the panoramic image. The performing the initial processing can include capturing a three-dimensional positioning of the anatomical structures of the panoramic image. The performing of the post-processing can include applying the automatic focusing process to only the jaw region or other subregion(s) within the jaw region and is performed on one or both of: a region-by-region basis, with each clinically relevant region containing two or more clinically relevant anatomical structures, and on a structure-by-structure basis. The performing of the post-processing can include reducing brightness of less relevant regions of the panoramic image outside the jaw region. Alternatively or additionally, the performing of the post-processing can include at least one of: contrast-enhancing a best one of the one or more clinically relevant anatomical structures, adjusting brightness of the jaw region, performing a noise reduction and contrast enhancement depending on the depth information and based upon a size, number or relative brightness of less clinically relevant regions of the panoramic image.

### Claims Related Examples

To further illustrate the devices and related methods disclosed herein, a non-limiting list of examples is provided below. Each of the following non-limiting examples can stand on its own, or can be combined in any permutation or combination with any one or more of the other examples.

Example 1 is a computer-implemented method comprising: performing an initial processing of an initial panoramic image derived from multiple x-ray projection images of a dental region of a patient; determining from the initial processing a depth information of anatomical structures of the panoramic image; identifying at least one of: a clinically relevant region of a jaw region and one or more clinically relevant anatomical structures of the jaw region of the panoramic image; performing a post-processing to at least one of: the clinically relevant region and the one or more clinically relevant anatomical structures using the depth information to enhance visualization of the at least one of the clinically relevant regions and the one or more clinically relevant anatomical structures of the panoramic image; and generating an updated panoramic image resulting from the performing the post-processing.

In Example 2, the subject matter of Example 1 includes, modifying post-processing parameters based on individual anatomical structures including one of the one or more clinically relevant structures and the clinically relevant region.

In Example 3, the subject matter of Examples 1-2 includes, wherein the one or more clinically relevant anatomical structures include teeth, root canals, root tips, tooth crowns, tooth pulps, and nerve canals.

In Example 4, the subject matter of Examples 1-3 includes, wherein performing the post-processing includes one or more of adjusting a contrast, performing a noise reduction, adjusting a brightness, adjusting a color information based on the depth information.

In Example 5, the subject matter of Examples 1-4 includes, wherein the performing the post-processing includes suppressing less clinically relevant regions of the panoramic image, wherein the less clinically relevant regions include one or more of: dental replacements, nasal cavities, sinuses and bone including an opposing jaw shadow and a spine.

In Example 6, the subject matter of Examples 1-5 includes, wherein performing the post-processing includes applying the depth information to only the jaw region or a subregion within the jaw region.

In Example 7, the subject matter of Examples 1-6 includes, wherein the performing the post-processing is performed on one or both of: a region-by-region basis, with each clinically relevant region containing two or more clinically relevant anatomical structures and on a structure-by-structure basis.

In Example 8, the subject matter of Examples 1-7 includes, displaying the panoramic image after post-processing on a display device for visual inspection by a dental practitioner.

In Example 9, the subject matter of Examples 1-8 includes, wherein identifying the at least one of the clinically relevant region and the one or more clinically relevant anatomical structures of the jaw region of the panoramic image includes identifying at least one of the anatomical structures that requires additional focusing to emphasize the anatomical structures.

In Example 10, the subject matter of Examples 1-9 includes, wherein performing post-processing includes reducing brightness of less clinically relevant regions and less clinically relevant structures.

In Example 11, the subject matter of Examples 1-10 includes, wherein performing post-processing includes contrast-enhancing a best one of the one or more clinically relevant anatomical structures, adjusting brightness of the jaw region depending on the depth information and performing brightness adjustment and a noise reduction based upon a size, number or relative brightness of less clinically relevant regions of the panoramic image.

Example 12 is a system for performing additional processing on an initial panoramic image derived from x-rays of a dental region of a patient, the system comprising: a processor; and a memory, in communication with the processor, with one or more instructions stored on the memory, the one or more instructions, when executed by the processor, cause the processor to perform operations comprising: perform with an automatic focusing process an initial processing of substantially an entirety of the initial panoramic image; determine from the initial processing a depth information of anatomical structures of the panoramic image; identify at least one of: a clinically relevant region of a jaw region and one or more clinically relevant anatomical structures of the jaw region of the panoramic image; perform a post-processing to the at least one of: the clinically relevant region and the one or more clinically relevant anatomical structures using the depth information to enhance visualization of the at least one of the clinically relevant region and the one or more clinically relevant anatomical structures of the panoramic image; and generate an updated panoramic image.

In Example 13, the subject matter of Example 12 includes, the post-processing includes applying the post-processing to only the jaw region.

In Example 14, the subject matter of Example 13 includes, wherein the post-processing includes contrast-enhancing one or more of the one or more clinically relevant anatomical structures or the clinically relevant region and adjusting brightness of the jaw region depending on the depth information.

In Example 15, the subject matter of Examples 12-14 includes, wherein the post-processing is applied to substantially the entirety of the panoramic image and includes at least one of: suppressing less clinically relevant regions of the panoramic image, reducing brightness of the less clinically relevant regions of the panoramic image outside the jaw region and performing a noise reduction based upon a size, number or relative brightness of the less clinically relevant regions of the panoramic image.

In Example 16, the subject matter of Examples 12-15 includes, wherein the post-processing includes one or more of adjusting a contrast, adjusting a color information, performing a noise reduction, and adjusting a brightness based on the depth information, wherein the one or more clinically relevant anatomical structures include teeth, root canals, root tips, tooth crowns, tooth pulps, and nerve canals, and wherein less clinically relevant regions include one or more of: dental replacements, nasal cavities, sinuses and bone including an opposing jaw shadow and a spine.

Example 17 is a non-transitory computer readable storage medium storing computer-readable instructions that when executed by a processor cause the processor to: initially process an initial panoramic image derived from x-rays of a dental region of a patient to determine a depth information of anatomical structures of the initial panoramic image; identify at least one of: a clinically relevant region of a jaw region and one or more clinically relevant anatomical structures of the jaw region of the panoramic image that requires additional focusing to emphasize the at least one of the anatomical structures; and perform a post-processing using the depth information to the at least one of: the clinically relevant region and the one or more clinically relevant anatomical structures to emphasize the one or more clinically relevant anatomical structures of the jaw region.

In Example 18, the subject matter of Example 17 includes, wherein the post-processing includes one or more of adjusting a contrast, performing a noise reduction, and adjusting a brightness based on the depth information, wherein the one or more clinically relevant anatomical structures include teeth, root canals, root tips, tooth crowns, tooth pulps, and nerve canals, and wherein less clinically relevant regions include one or more of: dental replacements, nasal cavities, sinuses and bone including an opposing jaw shadow and a spine.

In Example 19, the subject matter of Example 18 includes, wherein the post-processing includes contrast-enhancing a best one of the one or more clinically relevant anatomical structures, adjusting brightness of the jaw region depending on the depth information, suppressing the less clinically relevant regions of the panoramic image, reducing brightness of the less clinically relevant regions of the panoramic image outside the jaw region and performing the noise reduction based upon a size, number or relative brightness of the less clinically relevant regions of the panoramic image.

In Example 20, the subject matter of Examples 17-19 includes, display of the panoramic image after post-processing on a display device for visual inspection by a dental practitioner.

Example 21 is at least one machine-readable medium including instructions that, when executed by processing circuitry, cause the processing circuitry to perform operations to implement of any of Examples 1-20.

Example 22 is an apparatus comprising means to implement of any of Examples 1-20.

Example 23 is a system to implement of any of Examples 1-20.

Example 24 is a method to implement of any of Examples 1-20.

In the present detailed description, numerous specific details are set forth by way of examples to provide a thorough understanding of the relevant teachings. However, it should be apparent that the present teachings can be practiced without such details. In other instances, well-known methods, procedures, and/or components have been described at a relatively high level, without detail, to avoid unnecessarily obscuring aspects of the present teachings. The illustrative examples are described with respect to certain types of machines. The illustrative examples are also described with respect to other scenes, subjects, measurements, devices, data processing systems, environments, components, and applications only as examples. Any specific manifestations of these and other similar artifacts are not intended to be limiting to the disclosure. Any suitable manifestation of these and other similar artifacts can be selected within the scope of the illustrative examples. The illustrative examples can be implemented with respect to any type of data, data source, or access to a data source over a data network. Any type of data storage device can provide the data to an example of the disclosure, either locally at a data processing system or over a data network, within the scope of the disclosure. The illustrative examples can be used in conjunction with other comparable or similarly purposed structures, systems, applications, or architectures. For example, other comparable devices, structures, systems, applications, or architectures therefor, can be used in conjunction with such example of the disclosure within the scope of the disclosure. An illustrative example can be implemented in hardware, software, or a combination thereof.

The examples in this application are used only for the clarity of the description and are not limiting to the illustrative examples. Additional data, operations, actions, tasks, activities, and manipulations will be conceivable from this disclosure and the same are contemplated within the scope of the illustrative examples.

## Claims

1. A computer-implemented method comprising:
performing an initial processing of an initial panoramic image derived from multiple x-ray projection images of a dental region of a patient;
determining from the initial processing a depth information of anatomical structures of the panoramic image;
identifying at least one of: a clinically relevant region of a jaw region and one or more clinically relevant anatomical structures of the jaw region of the panoramic image;
performing a post-processing to at least one of: the clinically relevant region and the one or more clinically relevant anatomical structures using the depth information to enhance visualization of the at least one of the clinically relevant regions and the one or more clinically relevant anatomical structures of the panoramic image; and
generating an updated panoramic image resulting from the performing the post-processing.

2. The computer-implemented method of claim 1, further comprising modifying post-processing parameters based on individual anatomical structures including one of the one or more clinically relevant structures and the clinically relevant region.

3. The computer-implemented method of any one of claims 1-2, wherein the one or more clinically relevant anatomical structures include teeth, root canals, root tips, tooth crowns, tooth pulps, and nerve canals.

4. The computer-implemented method of any one of claims 1-3, wherein performing the post-processing includes one or more of adjusting a contrast, performing a noise reduction, adjusting a brightness, adjusting a color information based on the depth information.

5. The computer-implemented method of any one of claims 1-4, wherein the performing the post-processing includes suppressing less clinically relevant regions of the panoramic image, wherein the less clinically relevant regions include one or more of: dental replacements, nasal cavities, sinuses and bone including an opposing jaw shadow and a spine.

6. The computer-implemented method of any one of claims 1-5, wherein performing the post-processing includes applying the depth information to only the jaw region or a subregion within the jaw region.

7. The computer-implemented method of any one of claims 1-6, wherein the performing the post-processing is performed on one or both of: a region-by-region basis, with each clinically relevant region containing two or more clinically relevant anatomical structures and on a structure-by-structure basis.

8. The computer-implemented method of any one of claims 1-7, further comprising displaying the panoramic image after post-processing on a display device for visual inspection by a dental practitioner.

9. The computer-implemented method of any one of claims 1-8, wherein identifying the at least one of the clinically relevant region and the one or more clinically relevant anatomical structures of the jaw region of the panoramic image includes identifying at least one of the anatomical structures that requires additional focusing to emphasize the anatomical structures.

10. The computer-implemented method of any one of claims 1-9, wherein performing post-processing includes reducing brightness of less clinically relevant regions and less clinically relevant structures.

11. The computer-implemented method of any one of claims 1-10, wherein performing post-processing includes contrast-enhancing a best one of the one or more clinically relevant anatomical structures, adjusting brightness of the jaw region depending on the depth information and performing brightness adjustment and a noise reduction based upon a size, number or relative brightness of less clinically relevant regions of the panoramic image.

12. A system for performing additional processing on an initial panoramic image derived from x-rays of a dental region of a patient, the system comprising:
a processor; and
a memory, in communication with the processor, with one or more instructions stored on the memory, the one or more instructions, when executed by the processor, cause the processor to perform operations comprising:
perform with an automatic focusing process an initial processing of substantially an entirety of the initial panoramic image;
determine from the initial processing a depth information of anatomical structures of the panoramic image;
identify at least one of: a clinically relevant region of a jaw region and one or more clinically relevant anatomical structures of the jaw region of the panoramic image;
perform a post-processing to the at least one of: the clinically relevant region and the one or more clinically relevant anatomical structures using the depth information to enhance visualization of the at least one of the clinically relevant region and the one or more clinically relevant anatomical structures of the panoramic image; and
generate an updated panoramic image.

13. The system of claim 12, the post-processing includes applying the post-processing to only the jaw region, wherein the post-processing includes contrast-enhancing one or more of the one or more clinically relevant anatomical structures or the clinically relevant region and adjusting brightness of the jaw region depending on the depth information.

14. The system of any one of claims 12-13, wherein the post-processing is applied to substantially the entirety of the panoramic image and includes at least one of: suppressing less clinically relevant regions of the panoramic image, reducing brightness of the less clinically relevant regions of the panoramic image outside the jaw region and performing a noise reduction based upon a size, number or relative brightness of the less clinically relevant regions of the panoramic image.

15. The system of any one of claims 12-14, wherein the post-processing includes one or more of adjusting a contrast, adjusting a color information, performing a noise reduction, and adjusting a brightness based on the depth information, wherein the one or more clinically relevant anatomical structures include teeth, root canals, root tips, tooth crowns, tooth pulps, and nerve canals, and wherein less clinically relevant regions include one or more of: dental replacements, nasal cavities, sinuses and bone including an opposing jaw shadow and a spine.
